# EUROPEAN PATENT APPLICATION

(11) **EP 3 323 435 A1**
(43) Date of publication of application: **23.05.2018**
(21) Application number: 16199323.3
(22) Date of filing: 17.11.2016
(51) Int. Cl.: A61L 27/18, A61L 27/54, A61L 27/58

(54) **ACTIVE ENVELOPE SILICONE IMPLANT WITH DRUG SPACE AND MULTIPHASE MODE OF ACTION FOR BREAST AUGMENTATION**

(71) Applicant: Sorg, Heiko, 45147 Essen (DE); Steiert, Andreas, 10789 Berlin (DE)
(72) Inventor: Sorg, Heiko, 45147 Essen (DE); Steiert, Andreas, 10789 Berlin (DE)
(74) Representative: Hertin und Partner Rechts- und Patentanwälte PartG mbB

(57) **Abstract**

The invention relates to an implant comprising a shell filled with a polymeric gel and/or liquid that is covered with at least one bioresorbable envelope creating a space for a drug containing substance, such that the implant allows for a multiphase mode of action after implantation. The multiphase mode of action comprises a first phase relating to the function of the bioresorbable envelope and a second phase, wherein the resorption of the envelope leads to a release of the drug containing substance. The multiphase mode of the implant allows prevention of capsular contracture (CC) and supports the integration into the body pouch. The invention further relates to a method for the production of said implant as well as a kit comprising the implant and the drug containing substance.

## Description

The invention relates to an implant comprising a shell filled with a polymeric gel and/or liquid that is covered with at least one bioresorbable envelope creating a space for a drug containing substance, such that the implant allows for a multiphase mode of action after implantation. The multiphase mode of action comprises a first phase relating to the function of the bioresorbable envelope and a second phase, wherein the resorption of the envelope leads to a release of the drug containing substance. The multiphase mode of the implant allows prevention of capsular contracture (CC) and supports the integration into the body pouch. The invention further relates to a method for the production of said implant as well as a kit comprising the implant and the drug containing substance.

### BACKGROUND AND STATE OF THE ART

The most common implanted material in the human body consists of silicone. The implantation of silicone based implants for breast reconstruction or augmentation is still one of the most common performed procedures by reconstructive and aesthetic surgeons. Approximately 1.5 million units are sold per year by the world market leaders in the production of silicone breast implants. The main potential complications after silicone breast implantation have shown to be: (I) capsular fibrosis and contracture, (II) breast implant failure by rupture, distortion or leakage, (III) silicone gel bleeding and spreading throughout the organism, (IV) chronic inflammation around the silicone implant and (V) potential carcinogenicity of foreign implants.[1-6] The development of CC occurs with a rate of up to 80 % as shown in a review article by Berry and colleagues [7], and therefore displays a severe problem for patients. Hence CC formation displays the basis of many scientific studies in order to understand, prevent or treat its development. However the cause for CC formation is still not entirely clarified and seems to have a multifactorial genesis.

### Capsular contracture

In the past decades, millions of people have been exposed to various forms of silicones, which are temporarily or permanently implanted into the human body. Therefore, devices made of silicone are indispensible in the clinical routine. The chemical structure of silicone and many changes since its launch also raise many problems and questions on its local as well as systemic interactions. CC formation displays the result of a fibrotic foreign body reaction after implantation of silicone breast prostheses in the human body.[6,8-10] The physiological reaction of the body to the silicone implant forming a fibrous capsule has positive and negative effects. On the one hand the fibrous capsule stabilizes the positioning of the implant. On the other hand however CC is associated with pain, hardening, tightness, deformity and distortion of the breast.

Furthermore, capsular fibrosis is the number one reason for revision operations especially in implant exchange procedures, since capsular fibrosis does not occur as frequently in primary augmentations as in reconstruction/revision procedures.[11,12] The formation of a fibrous capsule is part of a reaction of the organism to the introduction of foreign material and takes place within one to two weeks postoperatively.[5] The myofibroblast displays one of the predominant cell types of the capsule, however, myofibroblasts are accompanied by macrophages, polymorphonuclear leukocytes, lymphocytes, plasma cells, and mast cells.[13-15] The capsule consists of approximately 27 % myofibroblasts with increasing tensile strength according to the degree of contracture.[16] Interestingly, this does not correlate with the number of fibroblasts.[16] The cellular source of the myofibroblast seems to be the bone marrow, as shown by Isom and colleagues, who found about 25 % of bone marrow derived stem cells in textured silicone shells in mice.[17] Prantl and colleagues further demonstrated that the capsular thickness was associated with the number of silicone particles and silicone-loaded macrophages in the peri-implant capsule and an increased local inflammatory reaction.[14,18] These histological findings, which were classified by the Wilflingseder score [19] correlated well to the clinical Baker classification. Additionally, it could be demonstrated that serum hyaluronan levels [13] as well as circulating immune complexes, procollagen III, anti-polymer antibodies and sICAM-1 [20] were significantly elevated in patients with CC, also correlating in part with the clinical Baker stage of contracture.[13] After multiple experimental and clinical trials there seems to be a consensus, that the use of textured outer shell surfaces, in comparison to smooth surfaces, are able to decrease the incidence of CC by disrupting contractile forces around the implant [12,21-26], emphasizing the need for better physical properties than cellular or pharmacological strategies of contracture formation.

A number of recent experimental studies have dealt with different issues thought to induce, prevent, reduce or treat capsular fibrosis and contracture around silicone breast implants.

### Immunological mechanisms

The criteria, which finally lead to integration or rejection of foreign material in the human organism are multi-faceted, however, immunological mechanisms play a major role in the development of CC after implantation of silicone breast implants. Therefore, the research on different immunological reactions and mechanisms is considered a starting-point for potential preventive strategies. Ojo-Amaize and colleagues in a blinded cross-sectional study demonstrated that women with symptomatic implanted as well as explanted silicone breast implants had twice as much abnormal silicone-specific T-cell responses compared to women with no symptoms. Further analysis demonstrated that these cells are CD4⁺, whereas the CD8⁺ T-cell population did not contribute to this activity.[27] The group of Ciapetti showed that peripheral blood lymphocytes of patients with silicone implants had a significantly increased rate of proliferation and viability when these cells have been re-exposed to silicone in vitro. Interestingly there was an even more significant difference between lymphocytes of aesthetic in comparison to reconstructive patients.[28] Two working groups, however, mainly contributed in detail on this issue. First, Smalley and coworkers confirmed, that patients with silicone implants are proned to an exaggerated T-cell mediated immune reactivity as given by an 18-fold higher stimulation index of stimulated lymphocytes of implant patients than of non-implant controls.[29] In an ongoing study Smalley further demonstrated, that the T-cell activation by silicone depends on monocytes.[30] The second group is lead by Dolores Wolfram, who studied the activity of immunological and inflammatory processes, which might be involved in CC formation, focusing on immune cells, proteins of the extracellular matrix, stress proteins and adhesion molecules.[31] The highest activity could be seen in the interface of the silicone implant and the formed fibrous capsule or in its direct adjacence, where activated CD4⁺ T-cells, macrophages and Langerhans-cell like dendritic cells could be detected. Furthermore, the tissue specimen showed intense positive staining of fibronectin on the interface, likely mediating interactions between the silicone and macrophages, fibroblasts and T-cells.[31] In order to determine the specificity and function of these T-lymphocytes, Wolfram and co-workers investigated the interplay of T-cells and their cytokine profile in capsular fibrosis. First, they verified that intracapsular lymphocytes were predominantly CD4⁺-cells producing a specific pro-fibrotic cytokine profile, which mediates the local immune response by means of activated TH1/TH17-cells. As the intracapsular T-cell ratio has been inversely proportional to the clinical stage of fibrosis, it has been hypothesized that pro-fibrotic cytokines and growth factors stimulate capsular fibrosis in the retention of local regulatory T-cells.[31,32]

### Biofilm in capsular contracture

Infections are the leading cause of morbidity after breast augmentation as proposed by Pittet et al.[6] Herein, the degree of CC seems to be associated with a prolonged or accelerated inflammatory process.[6,33-36] This inflammatory process might be exaggerated by local factors such as hematoma, infection or even by an invasion from remote-to-implant, which further increase the degree of inflammation finally leading to fibrosis.[37-40] The development of a septic biofilm at the time of implantation or within the first minutes to hours and the subclinical infection of the foreign material, especially with Stapyhlococcus epidermidis and Propionibacterium acnes, is described by many authors to have a high impact on the formation of CC.[6,37,38,41-43] The biofilm after silicone breast implantation refers to a certain liquid film resulting after the implantation of foreign material, which surrounds the respective device and can be described as a sterile or aseptic biofilm. This biofilm constitutes an interface between the foreign material and the organism, consisting of different chemoattractants and cytokines, which may either activate a foreign body response with inflammation or tolerate the material and integrate it into the body's homeostasis. Therefore the surface of the implanted material in close association with the sterile biofilm plays a major role in the determination of the reaction to the implant.

In the context of silicone breast implantation it should also be considered that the human breast region is not a sterile anatomic structure, as it has physiologic or even pathologic bacterial skin flora derived from the nipple ducts.[6] The highest incidence of capsular fibrosis seems to occur, when the silicone implant has been placed in a subglandular position via a periareolar approach through the mammary gland, leading to Staphylococcus epidermidis in the ductal tissue.[44] Additionally, it has been shown that the subclinical bacterial colonization plays a pivotal role in the development of high grade CC (Baker III/IV), as colonization could be detected in 66.7 % of explanted contractures, while low grade contractures (Baker I/II) did not show any colonization at all.[41] There is also a positive linear correlation between the severity of the contracture and the local inflammatory reaction.[41,45] To analyze this problem further, several authors and groups evaluated different experimental models and therapeutic strategies. Tamboto and colleagues developed a new in vivo pig model, in which female animals underwent augmentation mammaplasty using miniature gel-filled implants and pocket inoculation with Staphylococcus epidermidis.[46] The main outcome parameters have been the clinical Baker grade (tab. 1) and further laboratory testing of the resected capsules 13 weeks after implantation. This group could demonstrate that the presence of a septic biofilm results in subclinical infection and was associated with a four-fold increase of subsequent contracture formation. Interestingly, even non-inoculated pockets also developed contracture caused by the native porcine Staphylococcus epidermidis.[46] Without a doubt infection is a disruptive factor for the biocompatibility of silicone. The use of antibiotic substances is able to reduce the formation of CC as a unifactorial mechanism. In order to prevent bacterial colonization, several studies tried to administer local antiseptic washing or systemic antibiotics, however, with only minor effects.[38,47,48]

### Prophylactic strategies of CC

In order to prevent the formation of capsular fibrosis formation, several different approaches are possible. In the management of prophylactic strategies, the modulation of the implant surface texture and its patterning with different substances seems to be one promising approach to solve this problem.[49] To this end, the cell-to-surface interaction needs however to be better understood to engineer suitable materials for implantation. Using different microscopic techniques, Barr and co-workers investigated the cytoskeletal reaction of fibroblasts to silicone surfaces.[50] The results of this study revealed different possible reasons for the development of CC. It was shown that the smooth surface of implants predisposes the planar arrangement of the fibroblast around the implant. Interestingly, also the macroscopically smooth-surface implant presents with a rippled microscopic texture on the surface, which might increase the formation of a synovial type epithelium, which is experienced in fibrotic breast capsules.[51] Textured implant surfaces are able to decrease the formation of contracture, as the fibroblast anchors into the deep and random pattern, hence the fibroblast might not be able to align planarly.[51] Therefore, the authors concluded that cells might directly react to the topography of the biomaterial. In an ongoing study by Barr and co-workers the cytoskeletal reaction of breast tissue-derived human fibroblasts to silicone was investigated.[50] The authors could show that it is possible to create different topographies of silicone surfaces, which are able to induce a directional growth of fibroblasts as well as alterations in cell shape and their phenotype. The used fibroblasts have been able to detect the respective surface topography made of different width and depth of ridges, pits and pillars inducing contact guidance or orientation for the cells. The authors therefore concluded that the production of more biocompatible implants is possible via a change of the surface topography on which the fibroblasts might attach.

### OBJECTIVE OF THE INVENTION

The objective of the invention was to provide an implant that overcomes the disadvantages of implants of the prior art. In particular an objective of the invention was to provide an implant that is characterized by an augmented tolerance and integration in the human body minimizing the risk of CC formation.

### SUMMARY OF THE INVENTION

The objective is solved by the features of the independent claims. Preferred embodiments of the present invention are provided by the dependent claims.

The invention therefore relates to an implant suited for an implantation into the human body comprising a shell filled with a polymeric gel and/or liquid, wherein the shell is enveloped with at least one bioresorbable envelope such that at least one space is formed between the outer surface of the shell and the inner surface of the bioresorbable envelope and within said at least one space a substance is present comprising at least one drug with an immunosuppressive, antimicrobial and/or anti-inflammatory function.

Preferably the shell filled with a polymeric gel and/or liquid, refers to a breast implant as it is well known in the state of the prior art. The shell may therefore be for instance an elastomer silicon, preferably a vulcanized silicone rubber, which can be single or multi layered, smooth or textured, barrier-coated, or covered with polyurethane foam. Said shell is typically filled with a silicone gel or a saline solution. However also other fillings e.g. soy oil, polypropylene string etc. may be envisioned. The shape of the shell may have any conceivable shape, ranging from round, teardrop, contoured shapes or other preferred geometries. An average preferred breast implant may weigh between 50 to 1000 grams, or even more. Unlike state of the art breast implants the implant according to the invention however comprises in addition an envelope that covers said filled shell, preferably i.e. a state of the art breast implant. Furthermore between the envelope and the shell a space is left such that a drug containing substance may be inserted. Therefore upon implantation the initial reaction of the body will not be governed by the properties of the shell, but that of the envelope. Advantageously the envelope may therefore be functionalized to augment the integration process of the implant in a defined time span after surgery. For instance the envelope may comprise materials with antimicrobial properties in order to reduce the risk of infections after surgery.

Advantageously the envelope is bioresorbable that is the envelope comprise and/or consists of bioresorbable material which is absorbed by the body after implantation. Therefore antimicrobial, antiallergenic or other functions of the bioresorbable envelope, which may be unwanted in a subsequent long term integration process cede during the resorption phase of the envelope.

Furthermore during the resorption phase the envelope becomes permeable to the substance that is enclosed in the space between the inner surface of the envelope and the outer surface of the shell. It is particularly preferred that the substance comprises a drug and a pharmaceutically acceptable carrier for said drug. Preferably the substance is liquid at room temperature. After the resorption of the bioresorbable envelope the drug containing substance is released into the implant pocket. It is particularly preferred that the drug containing substance comprises an anti-inflammatory and immunosuppressive drug. It has been recognized by the inventors that CC formation after a breast implant is often associated with late onset of an immune reaction towards the implant resulting in inflammatory processes that give to the capsulation. Such inflammatory processes might be exaggerated by local factors such as hematoma, infection or even by an invasion from remote-to-implant. However with a suitable timing that coincides with the increase of the immune reaction the released anti-inflammatory and/or immunosuppressive drugs may advantageously dampen the deleterious effects of the immune response and counteract the formation of a CC.

The implant according to the invention therefore allows to control and support the integration of the implant in a multiphase mode. In the first phase the envelope governs the functionalization of the implant preferably within the first days or weeks after surgery. During the first phase it is paramount to ensure that infections and/or acute allergic reactions to the implant are prevented and the wound healing is supported. To this end the envelope is preferably functionalized to convey microbiological and/or antiallergenic properties. During this phase strong anti-inflammatory properties of the envelope may be counterproductive since they may reduce the function of the endogenous immune system. In the second phase, which preferably refers to the phase after the wound healing, the dominant risk during implantation relates to overreaction of the immune system towards the implant. In this second phase it is therefore desired to provide an efficient dose of anti-inflammatory and/or immunosuppressive drugs. These drugs may be accompanied by further antimicrobial drugs such as antibiotics to ensure that the suppression of the endogenous immune system does not lead to an increase of the pathogens and an infection. In a third phase, which corresponds to the phase after the drug containing substance is fully released and absorbed by the implant pocket, the shell of the implant is integrated into the body tissues. Advantageously the generation of the multiphase functionality of the implant, particularly comprising the first and the second phases, ensures that the long term integration of the shell of the implant occurs with reduced complications. The implant according to the invention therefore allows for a controlled multiphase integration process that significantly reduces the risk of CC formation and a long term stable and healthy integration of the implant, which is particularly advantageous for breast implants.

In a further preferred embodiment the invention relates to an implant suited for an implantation into the human body comprising a shell filled with a polymeric gel and/or liquid, wherein the implant is enveloped with a bioresorbable envelope such that a space is formed between the outer surface of the shell and the inner surface of the bioresorbable envelope, wherein the bioresorbable envelope exhibits an inlet for the injection of a substance, preferably comprising at least one drug with an immunosuppressive, antimicrobial and/or anti-inflammatory function.

The preferred embodiment of the invention relates to an implant suited for the introduction of a substance, preferably said substance encompassing a drug such as an anti-inflammatory, anti-microbial and/or immunosuppressive drug. To this end the implant comprises an inlet for an injection. It is preferred that the inlet only allows for a substance, preferably a liquid, to be injected into the space, whereas after the injection the inlet effectively prevents the substance to leak out of said space between the envelope and the shell. The inlet may for instance be a syringe inlet containing a valve. It may be particular preferred that the syringe inlet containing a valve is integrated into the envelope such that the outer surface of the envelope remains smooth without a disruption. It may also be preferred that after injecting the substance a part of the inlet may be removed in order to leave a smooth surface. In another preferred embodiment the inlet may relate to an envelope material suited for an injection of a substance with a syringe, wherein after the injection with the syringe the envelope is self-closing.

Advantageously the embodiment of an implant comprising an inlet allows for a particularly easy filling of the space between the envelope and the shell with a substance. Moreover due to the readily injectable substance advantageously the implant does not have to be produced and transported with the substance in said space. Therefore transport times can be longer and do not need specific cooling. Instead the implant may be transported as a "dry" implant, wherein the injection of the substance can occur shortly prior to the implantation of the implant and/or even in situ during the process of implantation. Moreover the implant with an inlet advantageously can be filled with different drug containing substances, thereby allowing for a specific tailoring of the drug to the needs of the subject, which will receive the implant. For instance the drug containing substance may be individually chosen for each subject, based upon its medical history, physiological condition and/or developed allergies. For instance an antibiotic drug may be chosen or discarded depending on previous reactions of the subject to said antibiotic drug. Also factors that may influence the reaction of the subject towards the implant may determine the choice of drug. By not having implants that are already filled with said drug, a flexible and individual optimization of the implant is possible in a particular cost-effective manner.

In a further preferred embodiment the invention relates to an implant wherein the bioresorbable envelope is biologically resorbed within 4 to 16 weeks, preferably 8 to 12 weeks. It is preferred that the time for the resorption of the envelope refers the resorption of the envelope to an extent at which the thickness of the envelope has diminished at least partially by at least 80 %, preferably at least 90 % most preferably 100 %. Moreover it is preferred that the time for the "resorption of the envelope" refers to the time at which sufficient material of the envelope of the body is resorbed such that the preferably at least 80 %, more preferably at least 90 % most preferably 100 % of the substance has leaked out of the space into the implant pocket. Therefore the time of the resorption of the envelope closely correlates with the time window, in which the substance containing the drug may convey its function to support the integration of the implant. This embodiment preferably allows for an immunosuppressive and/or anti-inflammatory drug to be released into the implant pocket between 4 to 16 weeks, preferably between 8 to 12 weeks after implantation. This time window coincides particular well with the onset of the risk of an increased immune response towards the implant, which for implants and methods of implantation in the prior art may result in CC. The preferred embodiment therefore allows for a particular effective counteraction of CC through a dosage of an immunosuppressive and/or anti-inflammatory drug in a therapeutically optimal time window.

A person skilled in the art knows how to design a biologically resorbable envelope that allows for a resorption within 4 to 16 weeks, preferably 8 to 12 weeks. To this end a combination of biologically resorbable material together with a specified thickness should be chosen, wherein a person skilled in the art may employ known resorptions tests in order to deduce suitable combinations.

In a further preferred embodiment the invention relates to an implant, wherein the bioresorbable envelope comprises a synthetic polymer and/or consists of a synthetic polymers preferably monocryl, polydioxanon and most preferably polylactide, polyglycolide or combinations of these. These materials have been proven to be particularly suited to allow for a multiphase support of the integration of the implant. The materials may support sufficiently well a strain that are applied on the envelope after integration of the implant, preferably the breast implant, into the human body. Therefore these materials reduce the risk of an unwanted rupture of the envelope leading to a premature leakage of the substance. Moreover the materials are well suited for the containment of the substance within said space throughout the first phase. Due to their chemical composition the materials are highly bioresorbable and can therefore be taken up by the human body without any health risks. During the resorption phase of said materials the envelope becomes permeable for the substance allowing for a well-controlled transition after a first phase into the second phase of the multiphase integration process. Moreover the materials are particularly biocompatible minimizing risks of a repulsive reaction after implantation. In addition the preferred materials exhibit a smooth outer surface and are acidic conveying further advantageous properties for the integration of the implant.

In a further preferred embodiment the invention relates to an implant wherein the bioresorbable envelope exhibits a thickness between 1 mm and 5 mm, preferably 2 mm and 4 mm. The preferred thickness of the envelope, in particular for envelopes consisting of the preferred synthetic polymers monocryl, polydioxanon and most preferably polylactide, polyglycolide or combinations of these, allow for a controlled and reliable resorption of the envelope within the preferred time span of 4 to 16 weeks, preferably 8 to 12 weeks.

In a further preferred embodiment the invention relates to an implant, wherein the bioresorbable envelope consists of and/or comprises a material with antimicrobial properties, preferably an acidic material, most preferably an acidic material exhibiting a pH value between 3 and 6. Due to the acidic property the bioresorbable envelope acts efficiently antimicrobial and bactericidal. This minimizes the concentration of pathogens in particular, bacteria, on the surface of the envelope and therefore impedes infections in the implant pocket. Moreover surprisingly, an acidic bioresorbable envelope, in particular for the preferred ranges of pH values accelerates the endogenous wound healing processes after surgery, further supporting the integration of the implant.

In a further preferred embodiment the invention relates to an implant, wherein the bioresorbable envelope exhibits an outer smooth surface. It has been recognized during the first phase, i.e. preferably the first days and weeks after implantation, it is immanent to prevent the spreading of pathogens on the surface of the implant. As described above acidic envelopes may already advantageously impede the proliferation of pathogens, in particular bacteria, on the surface of the envelope. However advantageously the mechanical properties of the envelope may also be constructed to reduce a risk of infection. By designing the envelope with an outer smooth surface bacteria cannot attach to the envelope, whereby local colonization of bacteria can be efficiently impeded. A smooth bioresorbable envelope may for instance be constructed by using materials such as the preferred synthetic polymers monocryl, polydioxanon, most preferably polylactide, polyglycolide or combinations of these.

In a further preferred embodiment the invention relates to an implant, wherein the at least one drug is an immunosuppressive glucocorticoid, preferably a halogenated glucocorticoid most preferably triamcinolone or dexamethasone. Using a substance that contains one of the preferred immunosuppressant has shown a particular pronounced therapeutic effect, which prevents an unwanted immune response to the implant and CC formation.

In a further preferred embodiment the invention relates to an implant, wherein the at least one drug is an antibiotic, preferably effective for gram-positive bacteria, most preferably selected from a group comprising penicillins, cephalosporins, lincosamids, carbapenemes and/or glycopeptides. Gram-positive bacteria have shown to constitute a threat for infection even at later stages of the integration process of an implant, in particular for breast implants. A drug that is an antibiotic may therefore efficiently reduce a spreading of said pathogens during the second phase of the integration process.

In a further preferred embodiment the invention relates to an implant, wherein the substance present in the space between the bioresorbable shell and the silicone shell comprises a combination of at least two different drugs in one and the same or different conditions, e.g. gel, liquid or powder. In this preferred embodiment a combination of different drugs may be chosen to ensure multiple therapeutic functions of the substance. It is particular preferred that one of the at least two drugs is an immunosuppressive and/or anti-inflammatory drug, while a second of the at least two drugs is an antimicrobial drug, preferably an antibiotic. Thereby during the second phase an immunosuppressive and/or anti-inflammatory drug may regulate the immune system in order to prevent the CC formation. Both substances classes lead to a down regulation of the immune system while the antimicrobial drug, preferably the antibiotic, ensures that the local downregulation of an endogenous immune response does not lead to the development of an infection.

In a further preferred embodiment the invention relates to an implant, wherein the outer surface of the silicon shell is textured. Textured surfaces of breast implants are well known in the prior art and designed to impede CC formation. Fibroblasts play a critical role during CC formation around breast implants. In particular on smooth surfaces of implants of the state of the art it has been shown that the fibroblasts predominantly adopt a planar arrangement around the implant. Textured surfaces decrease the formation of CC, as the fibroblast attach to irregularities in the pattern impeding a planar alignment. The preferred embodiment of a textured outer surface of the silicon shell therefore allows for the impediment of such an arrangement. A drawback of textured breast implants of the state of the art is however that the textured surface allows for an accumulation of pathogens e.g. bacteria within the pattern. This is due to the fact that the often textured surface that exhibit suitable patterns to impede a planar arrangement of fibroblast give rise to pockets in which body fluids are sequestered and which may serve as an incubation space for the bacteria. Advantageously using an implant as described herein during the first phase the implant is covered by the bioresorbable envelope, which exhibits preferably a smooth surface. Therefore bacterial growth, in particular at this sensitive phase after surgery, may be impeded. However during the second phase after several weeks, e.g. 4 to 6 weeks, the risk of inflammation ceases, while the risk of a formation of a fibrotic capsule due to the preferred anchorage of the fibroblasts rises. Advantageously at this time the envelope is resorbed such that the implant presents the textured surface to the body of the subject. This embodiment therefore allows for a multiphase supported integration of the implant not only due to the provision of a drug containing substance, but also due to a controlled timing of the structural properties of the surface of the implant that is presented to the surrounding tissues.

In a further preferred embodiment the invention relates to an implant, wherein the implant is a breast implant dimensioned and shaped for the reconstruction, reduction and/or augmentation of a female breast, preferably having a teardrop, round and/or contoured shape. The implant may preferably take any conceivable shape tailored to its desired function. The breast implant may be designed to allow for a reconstruction of a woman's breast that was affected by procedures such as mastectomy. Moreover the preferred breast implant can be used for cosmetic breast implantations to amend the appearance of a woman's breast, for example by adding an implant to increase the size of the breast, to correct asymmetries, change shape or fix deformities. To this end a person skilled in the art knows how to choose desired shape and/or size of breast implants. In a preferred embodiment a state of the art breast implant may be chosen, however said state of the art breast implant is covered by a bioresorbable envelope as described herein to construct a breast implant according to the invention.

In a further preferred embodiment the invention relates to a kit comprising a) an implant according to the invention or preferred embodiments thereof with a bioresorbable envelope that exhibits an inlet for the injection of a substance and b) a substance comprising at least one drug with an immunosuppressive, antimicrobial and/or anti-inflammatory function. Preferably the kit may additionally comprise a syringe or another suitable injection tool for the injection of the substance into the inlet. The kit therefore provided components in order to create an implant, comprising a shell filled with a polymeric gel and/or liquid, wherein the shell is enveloped with the bioresorbable envelope and wherein in the space between the envelope and the shell the substance is present comprising at least one drug with an immunosuppressive, antimicrobial and/or anti-inflammatory function. In order to arrive at such an implant it is preferably only necessary to inject the substance into the inlet. Advantageously by usage of a kit the implant does not have to be prefilled with the substance. Instead the substance may be injected into the implant shortly before or even after implantation. This facilitates the transportation of the implant. Furthermore in a preferred embodiment the kit may comprise at least two substances containing each at least one drug and/or a combination of at least two drugs. The kit may thus comprise a number of different substances. For instance the kit may comprise a first substance containing an immunosuppressive drug, e.g. triamcinolone, and an antibiotic, e.g. penicillin, and a second substance containing the same immunosuppressive drug, e.g. triamcinolone, but a different antibiotic e.g. cephalosporin. Depending on the known allergies of the subject that will receive the implant the substance is chosen and injected into the inlet.

In a further preferred embodiment the invention relates to a method for manufacturing an implant according to the invention and preferred embodiments thereof comprising the steps of
a) providing a shell filled with a polymeric gel and/or liquid
b) providing at least one bioresorbable envelope shaped to wrap the shell
c) wrapping the at least one bioresorbable envelope around the shell, whereby a space is created between the shell and the bioresorbable envelope
d) injecting a substance comprising at least one drug with an immunosuppressive, antimicrobial and/or anti-inflammatory function.

Such a method allows for a preparation of the implant that is cost-effective and easy to apply. A person skilled in the art understands that preferred embodiments and their advantageous effects that have been disclosed for the implant according to the invention equally apply to the kit according to the invention as well as the method for the production of the implant. For instance in a preferred embodiment of the implant an acidic bioresorbable envelope is disclosed, which reduces the risk of an infection after implantation. It is therefore obvious for a person skilled in the art that such an acidic bioresorbable envelope is also preferred for the kit according to the invention as well as for the method for a production of the implant.

### DETAILED DESCRIPTION OF THE INVENTION

All cited documents of the patent and non-patent literature are hereby incorporated by reference in their entirety.

As used herein, the term "envelope" preferably refers to a cover formed separately from a breast implant into which a breast implant may be inserted or a coating formed upon the surface of the breast implant. At times the terms "cover" and "coating" are used herein along with the generic term "covering," and unless a specific distinction is made between a cover and a coating, the details of the invention relating to coverings generally relate both to covers and coatings.

The term "bioresorbable", "biologically resorbable" or "resorbable" as used herein, means capable of being absorbed by the body.

As used herein, the term "body" preferably refers the organism or subject that receives the implant. Preferably the body is a vertebrate body, more preferably a mammal body, most preferably a human body. The term body is not limited to a particular age or sex.

As used herein, the term "implant pocket" or "implant pouch" preferably refers to the body part into which the implant is implanted. The term implant pocket or implant pouch therefore preferably refers to body tissues surrounding the implant after implantation.

The term "bioresorbable envelope" as used herein preferably refers to an envelope made of "bioresorbable polymer", which is preferably a polymer, which is degraded into a low molecular weight material through a degradation process such as a hydrolytic reaction or enzymatic reaction during the metabolism of an organism. In one embodiment, the bioresorbable polymer is polylactide (PLA), polyglycolide (PGA), poly(lactic-co-glycolic acid) (PLGA), polyorthoester, polyanhydride, polyamino acid, polyhydroxybutyric acid, polycaprolactone, polyalkylcarbonate, ethyl cellulose, chitosan, starch, guargum, gelatin, or collagen, but is not limited thereto. In a preferred embodiment the bioresorbable polymer is synthetic polymer preferably monocryl, polydioxanon and most preferably polylactide, polyglycolide or combinations of these.

As used herein, the term "drug" or "drugs" is used to include all types of therapeutic agents or pharmaceutical active substances, whether small molecules or large molecules such as proteins, nucleic acids and the like. The drugs of the invention can be used alone or in combination.

As used herein, "pharmaceutically acceptable carrier" includes any and all solvents, dispersion media, coatings, antibacterial, antiviral and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. Additionally, such pharmaceutically acceptable carriers can be aqueous or non-aqueous solutions, suspensions, and emulsions, most preferably aqueous solutions. Aqueous carriers include water, alcoholic/aqueous solutions, emulsions and suspensions, including saline and buffered media.

As used herein, the term "immunosuppressive drug," "immunosuppressive agent", "immunosuppressant" or "drug with an immunosuppressive function" refers to a drug that slows or halts immune system activity in a subject. Immunosuppressive agents can be given to a subject to prevent the subject's immune system from mounting an immune response after an organ transplant or for treating a disease that is caused by an overactive immune system. Examples of immunosuppressive agents include, but are not limited to, a calcineurin inhibitor, such as, but not limited to, cyclosporine, tacrolimus, a target of rapamycin (mTOR), such as, but not limited to, sirolimus, everolimus, zotarolimus, or temsorolimus, or an inhibitor of inosine monophosphate dehydrogenase, such as mycophenolate mofetil, an inhibitor of dihydrofolic acid reductase, such as, but not limited to, methotrexate, a corticosteroid, such as, but not limited to, prednisolone and methylprednisolone, or an immunosuppressive antimetabolite, such as, but not limited to, azathioprine. Particularly preferred immunosuppressive drugs are glucocorticoids, preferably a halogenated glucocorticoids most preferably triamcinolone or dexamethasone.

As used herein, the term "antimicrobial drug", "antimicrobial agent" or "drug with an antimicrobial function" preferably means any substance with which microbial pathogens are inactivated. Antimicrobial drugs include antibacterial, antiviral or antifungal agents, without being limited thereto. Antimicrobial agents also include substances that any combination of viricidal, bactericidal or fungicidal possess properties. Antimicrobial agents are those of ordinary skill are well known. Examples of antimicrobial agents include iodophors, iodine, benzoic acid, dehydroacetic acid, propionic acid, sorbic acid, methylparaben, ethylparaben, propylparaben, butylparaben, cetrimide, benzalkonium chloride, dequalinium chloride, chlorhexidine, chloreresol, chloroxylenol, benzyl alcohol, bronopol, chlorbutanol, ethanol, phenoxyethanol, phenylethyl alcohol, 2,4-dichlorobenzyl alcohol, thiomersal, clindamycin, erythromycin, benzoyl peroxide, mupirocin, bacitracin, polymyxin B, neomycin, triclosan, parachlormetaxylen, foscarnet, miconazole, fluconazole, itriconazol, ketoconazole, and pharmaceutically acceptable salts thereof.

As used herein, the term "antibiotic" preferably refers to a naturally-occurring substance produced by a microorganism, such as, without limitation, a fungus or a yeast, the substance being useful in the treatment of infectious disease and have been identified as possessing antibacterial, antifungal, antiviral, or antiparasitic activity.Antibiotic also refers to semi-synthetic substances wherein a molecular version produced by a microorganism is subsequently modified to achieve desired properties. Particularly preferred antibiotics are penicillins, cephalosporins, lincosamids, carbapenemes and/or glycopeptides. Moreover antibiotic may also refer to fully synthetic antibiotic which have been identified as possessing antibacterial, antifungal, antiviral, or antiparasitic activity. Examples of preferred synthetic antiobiotics include quinolones or oxazolidinones.

As used herein, the term "anti-inflammatory drug" refers to a drug that directly or indirectly reduces inflammation in a tissue. Examples of preferred anti-inflammatory drugs include nonsteriodial anti-inflammatrory drugs (NSAIDs such as acetylsalicylic acid, ibuprofen, paracetamol, metamizole, fenbuprofen, fenoprofen, flurbiprofen, indomethacin, ketoprofen or naproxen.

As used herein, the term "textured" or "texturized" preferably refers to a surface having minute indentations, deformations and/or raised portions on the subject surface. The textured surface has raised portions or indentations which are. The raised portions may be regularly or irregularly shaped. Also knitted, braided or fiber-like coatings such as Dacron velour, may be meant when referring to textured surfaces of the shell. Many commercially available breast implants include "textured surfaces" on the outer surface of the shell. Such textured surfaces are purposefully made to interact with the breast tissue in a healthy manner. Among other things, the type of texture may influence tissue ingrowth and reduce the occurrence of CC, an adverse event sometimes associated with breast implants.

As used herein, the term "outer" preferably refers to the side and/or location of a component of the implant in the direction towards the body pouch into which the implant is implanted. On the other hand as used herein, the term "inner" preferably refers to the side and/or location of a component of the implant in the direction away from the body pouch into which the implant is implanted, thus towards the interior center of the implant.

The term "biocompatible" as used herein refers to a property of a material that does not cause substantially harmful response to the subject when introduced to a subject. For example, it means that when materials or devices which are foreign to a subject are used, they do not induce substantially harmful reactions such as inflammatory reaction and/or immune reactions. Biocompatible materials which may be used for the present disclosure include biodegradable or biosafety materials.

The use of the terms "a" and "an" and "the" and similar referents in the context of describing the invention (especially in the context of the following claims) are to be construed to cover both the singular and the plural, unless otherwise indicated herein or clearly contradicted by context. As used herein, the term "a" includes at least one of an element that "a" precedes, for example, "a device" includes "at least one device." Further, it should further be noted that the terms "first," "second," and the like herein do not denote any order, quantity (such that more than one, two, or more than two of an element can be present), or importance, but rather are used to distinguish one element from another. The modifier "about" used in connection with a quantity is inclusive of the stated value and has the meaning dictated by the context (e.g., it includes the degree of error associated with measurement of the particular quantity).

All ranges disclosed herein are inclusive of the endpoints, and the endpoints are independently combinable with each other. The suffix "(s)" as used herein is intended to include both the singular and the plural of the term that it modifies, thereby including at least one of that term (e.g., the colorant(s) includes at least one colorants). "Optional" or "optionally" means that the subsequently described event or circumstance can or cannot occur, and that the description includes instances where the event occurs and instances where it does not. As used herein, "combination" is inclusive of blends, mixtures, alloys, reaction products, and the like.

### FIGURES

The following figures are presented in order to describe particular embodiments of the invention, by demonstrating a practical implementation of the invention, without being limiting to the scope of the invention or the concepts described herein.

### Short description of the figures:

- **Fig. 1:**: Schematic representation of a preferred embodiment of the implant
- **Fig. 2:**: Schematic representation of the space between the envelope and the shell of the preferred implant depicted in Fig. 1

### Detailed description of the figures:

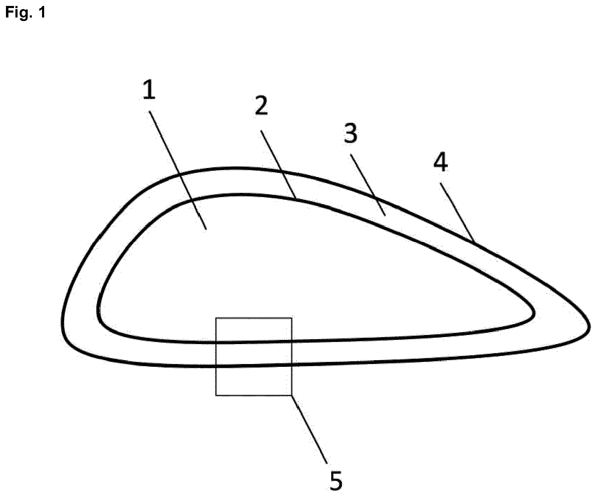

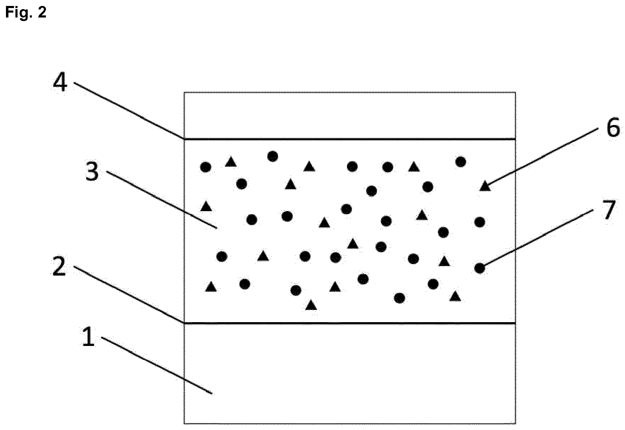

Fig. 1 is a schematic representation of a preferred embodiment of the implant. The implant is a breast implant and comprises a shell 2 that is filled with a polymeric gel and/or liquid 1. In a preferred embodiment the shell 2 is a textured silicon elastomer filled with a silicone gel and/or saline solution 1. The shell 2 filled with a liquid and/or polymeric gel 1 is preferably a breast implant as known in the state of the art. The shell 2 is enveloped by a bioresorbable envelope 4 thereby creating a space 3 in which a substance containing a drug 6 or 7 is present. Fig. 2 depicts the zoom region 5 indicated in Fig. 1 in order to illustrate the presents of the substance containing drugs 6 and/or 7 in the space 3.

The design of the implant enables a multiphase mode of action for the integration of the implant into a breast body pouch preventing CC formation. The multiphase mode of action starts after implantation of the breast implant.

In the 1^{st} phase after implantation the smooth bioresorbable envelope 4 has two different functions, which are achieved by preferred materials for the resorbable envelope material (see Example 1 for a list of particularly preferred materials). The preferred bioresorbable envelope 4 is purely synthetic and thus carries no residual risks, as is the case with biological products of animal or human origin. The degradation of the bioresorbable envelope and its degradation products further stimulate wound healing. The acidification of the wound pouch by the preferred biological resorbable material additionally acts bactericidal, thereby minimizing the risk of infection and reducing the immune response of the body. Moreover the acidic pH value accelerates the wound healing process, which further supports the integration. Due to these properties the bioresorbable envelope minimizes the rate of wound infections directly after implantation of the device and reduces the risk of allergization.

For the preferred embodiment on the one hand bacteria may not attach to the smooth envelope and on the other hand the envelope may act by local anti-inflammatory effects.

After resorption of the envelope the space 3 will get opened, releasing the prefilled substances containing drugs 6 and/or 7. Preferred examples for the drugs are listed in Example 2. After resorption of the envelope 4 therefore a 2^{nd} phase of the multiphase mode of action is started which comprises the release of immunosuppressive and/or anti-inflammatory drugs in order to reduce CC formation. The space 2 may get individually prefilled with single drugs or combination of drugs, preferably according to Example 2, in particular with anti-inflammatory and/or immunosuppressive drugs.

In the last phase, that is the 3^{rd} phase the textured shell 2 filled with a polymeric gel and/or liquid 1 is integrated in the pectoral pouch. The different phases reduce or prevent CC formation by shielding a state of the art silicone implant surface, i.e. the shell 2, by different synchronized mechanisms as long as possible from the host immune system. Thereby the implant may be integrated in the 3^{rd} phase without harmful complications.

### EXAMPLES

The invention is further described by the following examples. These are not intended to limit the scope of the invention.

### Example 1

| **Highly preferred** | **Preferred** |
|---|---|
| polyactic acid | monocryl |
| | polydioxanone |

Example 1 summarizes preferred embodiments for bioresorbable materials for the bioresorbable envelope. The preferred materials are ordered for their preferential use in the implant according to the invention based upon experimental data on their biocompatibility, bioresorbability as well as acidic properties in order to allow for an antimicrobial function of the envelope.

### Example 2

| **Highly preferred** | **Preferred** |
|---|---|
| triamcinolone | penicillines (e.g. amoxicillin) |
| | cephalosporins |
| | lincosamides |
| | glycopeptides |

Example 2 summarizes preferred embodiments for drugs that may be contained in the substance between the bioresorbable envelope and the outer surface of the shell. The drugs are ordered according to their preferential use for preferred embodiments of the implant based upon experimental data on their immunosuppressive, anti-inflammatory and/or antimicrobial function in order to reduce the risk of CC formation.

### LITERATURE

[1] Gerszten PC. A formal risk assessment of silicone breast implants. Biomaterials 1999;20:1063-9.
[2] Gampper TJ, Khoury H, Gottlieb W, et al. Silicone Gel Implants in Breast Augmentation and Reconstruction. Annals of Plastic Surgery 2007;59:581-90. doi:10.1097/01.sap.0000258970.31562.5d.
[3] Hajdu SD, Agmon-Levin N, Shoenfeld Y. Silicone and autoimmunity. European Journal of Clinical Investigation 2010;41:203-11. doi:10.1111/j.1365-2362.2010.02389.x.
[4] Zuckerman DM. Reasonably safe? Breast implants and informed consent. Reprod Health Matters 2010;18:94-102. doi:10.1016/S0968-8080(10)35520-0.
[5] Goldberg EP. Silicone breast implant safety: physical, chemical, and biologic problems. Plast Reconstr Surg 1997;99:258-61.
[6] Pittet B, Montandon D, Pittet D. Infection in breast implants. The Lancet Infectious Diseases 2005;5:94-106. doi:10.1016/S1473-3099(05)01281-8.
[7] Berry MG, Cucchiara V, Davies DM. Breast augmentation: Part II - adverse capsular contracture. J Plast Reconstr Aesthet Surg 2010;63:2098-107. doi:10.1016/j.bjps.2010.04.011.
[8] Embrey M, Adams EE, Cunningham B. Factors associated with breast implant rupture: pilot of a retrospective analysis. Aesthetic Plast Surg 1999;23:207-12.
[9] Iwuagwu F. Silicone breast implants: complications. British Journal of Plastic Surgery 1997.
[10] Schaub TA, Ahmad J, Rohrich RJ. Capsular contracture with breast implants in the cosmetic patient: saline versus silicone--a systematic review of the literature. Plast Reconstr Surg 2010;126:2140-9. doi:10.1097/PRS.0b013e3181f2b5a2.
[11] Handel N, Cordray T, Gutierrez J. A long-term study of outcomes, complications, and patient satisfaction with breast implants. Plast Reconstr Surg 2006;117:757-67-discussion768-72. doi:10.1097/01.prs.0000201457.00772.1d.
[12] Handel N, El-Komy MH, Jensen JA, Schmidt RL, Black Q, Widness JA, et al. The fate of breast implants: a critical analysis of complications and outcomes. Plast Reconstr Surg 1995;96:1521-33.
[13] Prantl L, Pöppl N, Horvat N. Serologic and histologic findings in patients with capsular contracture after breast augmentation with smooth silicone gel implants: is serum hyaluronan a potential predictor? Aesthetic Plast Surg 2005;29:510-8. doi: 10.1007/s00266-005-5049-y.
[14] Prantl L, Schreml S, Fichtner-Feigl S, et al. Clinical and morphological conditions in capsular contracture formed around silicone breast implants. Plast Reconstr Surg 2007;120:275-84. doi:10.1097/01.prs.0000264398.85652.9a.
[15] Coleman DJ, Sharpe DT, Naylor IL, et al. The role of the contractile fibroblast in the capsules around tissue expanders and implants. British Journal of Plastic Surgery 1993;46:547-56.
[16] Hwang K, Sim HB, Huan F, Kim DJ. Myofibroblasts and Capsular Tissue Tension in Breast Capsular Contracture. Aesthetic Plast Surg 2010;34:716-21. doi:10.1007/s00266-010-9532-8.
[17] Isom C, Kapoor V, Wilson L, Fathke C, Barnes L, Sullivan SR, et al. Breast Implant Capsules Are Partially Composed of Bone Marrow Derived Cells. Annals of Plastic Surgery 2007;58:377-80. doi:10.1097/01.sap.0000243996.37786.4a.
[18] Prantl L, Schreml S, Fichtner-Feigl S, et al. Histological and immunohistochemical investigations with capsular contracture after breast augmentation with smooth silicone gel implants. Handchir Mikrochir Plast Chir 2006;38:224-32. doi:10.1055/s-2006-924420.
[19] Wilflingseder P, Hoinkes G, Mikuz G. Tissue reactions from silicone implant in augmentation mammaplasties. Minerva Chir 1983;38:877-80.
[20] Wolfram D, Oberreiter B, Mayerl C, et al. Altered systemic serologic parameters in patients with silicone mammary implants. Immunology Letters 2008;118:96-100. doi:10.1016/j.imlet.2008.03.007.
[21] Ulrich D, Ulrich F, Pallua N, Eisenmann-Klein M. Effect of tissue inhibitors of metalloproteinases and matrix metalloproteinases on capsular formation around smooth and textured silicone gel implants. Aesthetic Plast Surg 2009;33:555-62. doi: 10.1007/s00266-009-9335-y.
[22] Collis N, Coleman D, Foo IT, et al. Ten-year review of a prospective randomized controlled trial of textured versus smooth subglandular silicone gel breast implants. Plast Reconstr Surg 2000;106:786-91.
[23] Wong C-H, Samuel M, Tan B-K, et al. Capsular Contracture in Subglandular Breast Augmentation with Textured versus Smooth Breast Implants: A Systematic Review. Plast Reconstr Surg 2006;118:1224-36. doi:10.1097/01.prs.0000237013.50283.d2.
[24] Malata CM, Feldberg L, Coleman DJ, Foo IT, Sharpe DT. Textured or smooth implants for breast augmentation? Three year follow-up of a prospective randomised controlled trial. British Journal of Plastic Surgery 1997;50:99-105.
[25] Coleman DJ, Foo ITH, Sharpe DT. Textured or smooth implants for breast augmentation? A prospective controlled trial. British Journal of Plastic Surgery 1991 ;44:444-8. doi:10.1016/0007-1226(91)90204-W.
[26] Hakelius L, Ohlsén L. Tendency to capsular contracture around smooth and textured gel-filled silicone mammary implants: a five-year follow-up. Plast Reconstr Surg 1997;100:1566-9.
[27] Ojo-Amaize EA, Wong C-H, Conte V, Samuel M, Lin HC, Tan B-K, et al. Silicone-specific blood lymphocyte response in women with silicone breast implants. Clin Diagn Lab Immunol 1994;1:689-95.
[28] Ciapetti G, Granchi D, Stea S, et al. Assessment of viability and proliferation of in vivo silicone-primed lymphocytes after in vitro re-exposure to silicone. J Biomed Mater Res 1995;29:583-90. doi: 10.1002/jbm.820290505.
[29] Smalley DL, Shanklin DR, Hall MF, Stevens MV, Hanissian A. Immunologic stimulation of T lymphocytes by silica after use of silicone mammary implants. Faseb J 1995;9:424-7.
[30] Smalley DL, Shanklin DR, Hall MF. Monocyte-dependent stimulation of human T cells by silicon dioxide. Pathobiology 1998;66:302-5.
[31] Wolfram D, Dolores W, Rainer C, et al. Cellular and molecular composition of fibrous capsules formed around silicone breast implants with special focus on local immune reactions. J Autoimmun 2004;23:81-91. doi:10.1016/j.jaut.2004.03.005.
[32] Momoh AO, Chung KC. Discussion. Plast Reconstr Surg 2012;129:338e-339e. doi:10.1097/PRS.0b013e3182406fe3.
[33] Khan UD. Breast Augmentation, Antibiotic Prophylaxis, and Infection: Comparative Analysis of 1,628 Primary Augmentation Mammoplasties Assessing the Role and Efficacy of Antibiotics Prophylaxis Duration. Aesthetic Plast Surg 2009;34:42-7. doi: 10.1007/s00266-009-9427-8.
[34] Burkhardt BR, Losken A, Fried M, Carlson GW, Schnur PL, Culbertson JH, et al. Capsules, infection, and intraluminal antibiotics. Plast Reconstr Surg 1981;68:43-9.
[35] De Cholnoky. Augmentation mammaplasty. Survey of complications in 10,941 patients by 265 surgeons. Plast Reconstr Surg 1970;45:573-7.
[36] Cronin TD, Greenberg RL. Our experiences with the silastic gel breast prosthesis. Plast Reconstr Surg 1970;46:1-7.
[37] Virden CP, Dobke MK, Stein P, et al. Subclinical infection of the silicone breast implant surface as a possible cause of capsular contracture. Aesthetic Plast Surg 1992;16:173-9.
[38] van Heerden J, Turner M, Hoffmann D, et al. PRAS851_1679_proof 610..617. J Plast Reconstr Aesthet Surg 2009;62:610-7. doi:10.1016/j.bjps.2007.09.044.
[39] Deva A, Chang L. Bacterial Biofilms: A Cause for Accelerated Capsular Contracture? Aesthetic Surgery Journal 1999;19:130-3. doi:10.1053/aq.1999.v19.97038.
[40] Shah Z, Lehman JA, Tan J. Does infection play a role in breast capsular contracture? Plast Reconstr Surg 1981;68:34-42.
[41] Schreml S, Poeppl N, Heine N, Schreml S, Eisenmann-Klein M, Lichtenegger F, et al. Bacterial Colonization Is of Major Relevance for High-Grade Capsular Contracture After Augmentation Mammaplasty. Annals of Plastic Surgery 2007;59:126-30. doi:10.1097/01.sap.0000252714.72161.4a.
[42] Pajkos A, Deva AK, Vickery K, et al. Detection of Subclinical Infection in Significant Breast Implant Capsules. Plast Reconstr Surg 2003;111:1605-11. doi:10.1097/01.PRS.0000054768.14922.44.
[43] Macadam SA, Clugston PA, Germann ET. Retrospective Case Review of Capsular Contracture After Two-Stage Breast Reconstruction. Annals of Plastic Surgery 2004;53:420-4. doi:10.1097/01.sap.0000130705.19174.d4.
[44] Dobke MK, Vastine VL, et al. Characterization of microbial presence at the surface of silicone mammary implants. Annals of Plastic Surgery 1995;34:563-9-disscusion570-1.
[45] Poeppl N, Schreml S, Lichtenegger F, et al. Does the surface structure of implants have an impact on the formation of a capsular contracture? Aesthetic Plast Surg 2007;31:133-9. doi:10.1007/s00266-006-0091-y.
[46] Tamboto H, Vickery K, Deva AK. Subclinical (Biofilm) Infection Causes Capsular Contracture in a Porcine Model following Augmentation Mammaplasty. Plast Reconstr Surg 2010;126:835-42. doi:10.1097/PRS.0b013e3181e3b456.
[47] Rennekampff HO, Exner K, Lemperle G, Nemsmann B. Reduction of capsular formation around silicone breast implants by D-penicillamine in rats. Scand J Plast Reconstr Surg Hand Surg 1992;26:253-5.
[48] Unlu RE, Yilmaz AD, Orbay H, et al. Influence of Rifampin on Capsule Formation Around Silicone Implants in a Rat Model. Aesthetic Plast Surg 2007;31:358-64. doi: 10.1007/s00266-006-0248-8.
[49] Steiert A, Reimers K, Burke W, Zapf A, Vogt P. Covalent vectored binding of functional proteins by bifunctional crosslinking at silicone interfaces. J Biomed Mater Res 2012;100A:1248-55. doi:10.1002/jbm.a.34008.
[50] Barr S, Hill E, Bayat A. Patterning of novel breast implant surfaces by enhancing silicone biocompatibility, using biomimetic topographies. Eplasty 2010;10:e31.
[51] Barr S, Hill E, Bayat A. Current implant surface technology: an examination of their nanostructure and their influence on fibroblast alignment and biocompatibility. Eplasty 2009;9:e22.

### LIST OF REFERENCE CHARACTERS

- 1: polymeric gel and/or liquid filling of the shell
- 2: shell
- 3: space for the substance
- 4: bioresorbable envelope
- 5: zoom region on the space 2
- 6: first drug
- 7: second drug

## Claims

1. Implant suited for an implantation into the human body comprising a shell (2) filled with a polymeric gel and/or liquid (1)
**characterized in that**
the shell (2) is enveloped with at least one bioresorbable envelope (4), such that at least one space (3) is formed between the outer surface of the shell (2) and the inner surface of the bioresorbable envelope (4) and
within said at least one space a substance is present comprising at least one drug (6,7) with an immunosuppressive, antimicrobial and/or anti-inflammatory function.

2. Implant suited for an implantation into the human body comprising a shell (2) filled with a polymeric gel and/or liquid (1)
**characterized in that**
the implant is enveloped with a bioresorbable envelope (4), such that a space (3) is formed between the outer surface of the shell (2) and the inner surface of the bioresorbable envelope (4), wherein the bioresorbable envelope (4) exhibits an inlet for the injection of a substance.

3. Implant according to the any one of the preceding claims **characterized in that**
the bioresorbable envelope (4) is biologically resorbed within 4 to 16 weeks, preferably 8 to 12 weeks.

4. Implant according to any one of the preceding claims **characterized in that**
the bioresorbable envelope (4) comprises a synthetic polymer preferably monocryl, polydioxanon and most preferably polylactide, polyglycolide or combinations of these.

5. Implant according to any one of the preceding claims **characterized in that**
the bioresorbable envelope (4) exhibits a thickness between 1 mm and 5 mm, preferably 2 mm and 4 mm.

6. Implant according to any one of the preceding claims **characterized in that**
the bioresorbable envelope (4) comprises a material with antimicrobial properties, preferably an acidic material, most preferably an acidic material exhibiting a pH value between 3 and 6.

7. Implant according to any one of the preceding claims
**characterized in that**
the bioresorbable envelope (4) exhibits an outer smooth surface.

8. Implant according to any one of the preceding claims
**characterized in that**
the at least one drug (6, 7) is an immunosuppressive glucocorticoid, preferably a halogenated glucocorticoid most preferably triamcinolone or dexamethasone.

9. Implant according to any one of the preceding claims
**characterized in that**
the at least one drug (6, 7) is an antibiotic, preferably effective for Gram-positive bacteria, most preferably selected from a group comprising penicillins, cephalosporins, lincosamids, carbapenemes and/or glycopeptides.

10. Implant according to any one of the preceding claims
**characterized in that**
the substance present in the space (3) between the bioresorbable envelope (4) and the shell (2) comprises a combination of at least two different drugs (6, 7).

11. Implant according to any one of the preceding claims
**characterized in that**
the outer surface of the shell (2) is textured.

12. Implant according to any one of the preceding claims
**characterized in that**
the implant is a breast implant dimensioned and shaped for the reconstruction, reduction and/or augmentation of a female breast, preferably having a teardrop, round and/or contoured shape.

13. Kit
comprising
a) an implant according to any one of claims 2 - 12, wherein the bioresorbable envelope (4) exhibits an inlet for the injection of a substance and
b) a substance comprising at least one drug (6, 7) with an immunosuppressive, antimicrobial and/or anti-inflammatory function.

14. Method for manufacturing an implant according to any one of the preceding claims 2 - 12 comprising the steps of
a) providing a shell (2) filled with a polymeric gel and/or liquid (1)
b) providing at least one bioresorbable envelope (4) shaped to wrap the shell (2)
c) wrapping the at least one bioresorbable envelope (4) around the shell (2), whereby a space (3) is created between the shell (2) and the bioresorbable envelope (4)
d) injecting a substance comprising at least one drug (6, 7) with an immunosuppressive, antimicrobial and/or anti-inflammatory function.
